# EUROPEAN PATENT SPECIFICATION

(11) **EP 1 516 596 B1**
(45) Date of publication and mention of the grant of the patent: **22.07.2009**
(21) Application number: 03709837.3
(22) Date of filing: 18.03.2003
(51) Int. Cl.: A61F 2/00, A61B 17/04

(54) **IMPROVED SYSTEM FOR THE TREATMENT OF STRESS URINARY INCONTINENCE**
VERBESSERTES SYSTEM ZUR BEHANDLUNG DER STRESSBEDINGTEN HARNINKONTINENZ
SYSTEME AMELIORE DESTINE AU TRAITEMENT DE L'INCONTINENCE URINAIRE D'EFFORT

(30) Priority: 29.05.2002 ES 200201227
(43) Date of publication of application: 23.03.2005
(73) Proprietor: Romero Maroto, Jesus, 03560 El Campello (Alicante) (ES)
(72) Inventor: Romero Maroto, Jesus, 03560 El Campello (Alicante) (ES)
(74) Representative: Sugrañes Patentes y Marcas
(86) International application number: PCT/ES2003/000128
(87) International publication number: WO 2003/101344

(56) References cited:
- EP-A- 1 552 796
- WO-A-02/26108
- WO-A-03/092546
- WO-A2-00/74633
- WO-A2-02/28314
- FR-A1- 2 787 990
- FR-A1- 2 814 939

## Description

### Object of the invention

The present invention relates to an improved system for the treatment of stress urinary incontinence, which provides essential characteristics of novelty and appreciable advantages in relation to the known means used for the same purposes in the current state of the art.

More particularly, the system proposed by the invention develops means especially indicated for the treatment of said pathology, which appreciably improves the application of the techniques used at present, especially that known as the sling technique, using for this elements which, being of a similar conception to conventional ones, have been advantageously modified and simplified. Said means include a single thread passer, of a smaller calibre than those currently known, provided with an orifice in its distal end which permits the holding and subsequent pulling of the mesh so that each one of its ends terminates in two threads which may be threaded through the eye of the needle, to perform the positioning of the mesh by pulling, said mesh also having the advantageous modification of having being provided with small holes which permit threads, intended to facilitate the pulling operation and that of tightening/loosening the mesh once positioned, to pass through.

The field of application of the invention is in the industrial sector dedicated to the manufacturing of utensils, instruments and devices for medical use, and in particular medical-surgical use.

### Background and Summary of the Invention

The fact that stress urinary incontinence is a frequent pathology in women is generally known, consisting of urine loss when abdominal stress is performed such as coughing, going from the sitting position to that of standing up or walking.

Said problem constitutes an illness which frequently causes a serious reduction in the quality of life, as a consequence of the voluntary limitation of multiple activities, due to fear and shame that said loss may be noted by other people, and which often leads to situations of loneliness and isolation.

The anatomic alteration which lies behind this urine loss symptomology, consists of the descent of the vesical neck and the urethra, due to the alteration of the perineal muscles, caused, amongst other reasons, due to giving birth. This vesical neck and urethra descent means that abdominal pressures are not correctly transmitted thereto, this set of altered pressures being the cause of the involuntary urine leaks.

Until now, some techniques have been proposed which tend to provide solutions to the problem posed. In any case, the solution to said problem of incontinence is surgical and consists of returning the vesical neck and urethra to their original position, then providing them with a support which permits the correct transmission of abdominal pressures thereto. Amongst the techniques used, that known as sling is the one which provides a more efficient, lasting solution.

This sling technique consists of placing a tissue band, whether from the patient herself or heterologous tissue (marlex, silicone and, lately and with best results, prolene), in a sub-cervical or sub-urethral position, so that once said band is fixed in different points according to the technique, it lifts and supports the vesical neck and the urethra in their original position, thus enabling pressure transmission and avoiding urine loss.

The tissue band can be sufficiently long to go from the urethra to the hypogastrium, or it can consist only of a small patch joined to 4 threads, which are later attached to the abdominal wall.

The aggressiveness of the technique has been considerably reduced since it is performed intravaginally, without needing to open the pelvic cavity, and since prolene has been used, as it avoids having to collect aponeurosis from the same patient.

Recently, within the concept of this surgery as non-invasive, two sets have come onto the market for urinary incontinence surgery, identified by the acronyms TVT® and SPARC®. Both consist of a polypropylene mesh, surrounded by a plastic cover, and a connector system consisting of a needle with a handle. Both techniques are differentiated in that, in the case of TVT®, the needle is passed through the vagina towards the hypogastrium, needing to separate the urethra with a probe and a catch, whilst in the case of the SPARC® technique, this step is performed in the direction of hypogastrium to the vagina, without the urethra needing to be separated by a probe. In both cases, the polypropylene mesh used, surrounded by a plastic cover, is passed through the vagina to the hypogastrium, which has facilitated the mesh passing through, leaving the latter positioned subcutaneously, where it is fixed due to the properties it has been provided with.

In both techniques, and all those wherein the sling system is used, the most important problem arising consists of the tension given to the sling being correctly adjusted, being a special feature that conditions the success or failure of this type of surgery. Furthermore, if it is subjected to excessive tension, a urinary obstruction may occur, so that the patient will have difficulties in eliminating urine. In contrast, if sufficient tension is not provided, the patient will then continue to have an incontinence problem.

There is, therefore, a practical difficulty associated with the fact that there is no way of calculating the most appropriate tension, having to do so randomly and approximately. Once the operation has concluded, it is then not possible to correct the excess or lack of tension, which leads to a failure rate estimated at between 10% and 20% of the operations. Furthermore, this problem is the cause of all failures which occur with the application of this technique.

The existence of a system identified as REEMEX® is also known in the market, wherein a subcutaneous implant of a device in the form of a pulley is performed in the hypogastrium, which permits, with the use of a screwdriver, and during the postoperative, to increase the tension given to a sling formed by a small patch of tissue fastened with four threads. This system is expensive, complex and suffers from considerable potential complications, due to the fact that it requires the implantation of a foreign body. It also can be loosened if the sling has been made with threads, but the system cannot be used when it uses a sling only formed from mesh, i.e. of TVT® or SPARC® type.

Some of these techniques are described, for example, in further detail in the following patent documents: WO 0074633 and WO 0226108.

WO 0074633 discloses a surgical instrument that comprises a needle-like element that attaches to a mesh tape. The distal end of the needle element terminates at a conical section having a tip, like a blunt. There is a handle attached to the needle. The needle includes tying the ends of the tape to form a knot and securely inserting the knot into the V-type groove in shaft. Not only is this instrument very complicated, it also involves using two needle elements for implanting the tape into the lower abdomen of a female.

WO 0226108 discloses a pair of curved delivery needles, each one defining a distal end a proximal end and adopted to be inserted into the abdomen of a female and to be positioned on either side of the bladder neck, so as to define a delivery path for a tape, which may be removably attached to the proximal ends of the delivery needles through the vagina for implantation into the abdomen, in order to provide support for the urethra. This assembly comprises two parts, one internal needle and one external scabbard, across which the mesh is extracted with an elaborated system of union with the needle.

Finally, there is another system currently in existence, although there is no knowledge of its commercialization, consisting of a development by Dr. Gil Vemet, which includes a liquid reservoir implanted in subcutaneous tissue in the hypogastrium. The threads which fasten the sling have been attached to said reservoir. The system allows, if there is little tension, that the reservoir can be filled with liquid, thereby increasing the tension. The system is of equally difficult clinical application, and cannot be used with complete meshes.

In accordance with the previous description, the systems used in the application of the sling technique have a series of drawbacks associated with their use, which do not permit the fully satisfactory use thereof.

The present invention has proposed the main objective of developing a system whereby an efficient, quick, economic solution is provided to said problems of the current art, especially that regarding the application of the sling system.

This objective has been fully attained by the system of the invention, wherein a mesh positioning device (or thread passer) and a mesh, intervene as fundamental elements.

As regards the thread passer, it has the advantage that it is of smaller calibre than those currently used, meaning it is less aggressive, with a considerable reduction in the possibilities of causing iatrogenic lesions; furthermore, the thread passer is universal, i.e. it serves for both directions, being able to pass from vagina to hypogastrium, or from hypogastrium to vagina; also, regarding the connection system of the mesh to thread passer, it has been considerably simplified, as a hole in the distal part of the needle permits threading the end part of the mesh formed by threads.

As regards the mesh provided for the invention for its application with a view to maintaining the vesical neck and the urethra in an optimum position, it has certain advantageous characteristics in comparison with those used at present, amongst which we can be state, for example, the fact that it has no additional cover requires no withdrawal or extraction period as occurs at present, thus simplifying the procedure; furthermore, the mesh terminates in a reduction at the ends to two threads, which are those which are threaded in the thread passer and achieve the positioning of the mesh by pulling; finally, it should also be stated, as a particularly important characteristic, the fact that the mesh has holes distributed thereon, in predetermined positions, once the mesh has been positioned, on a aponeurosis of the rectus level and periurethral level, allowing threads to be introduced in said holes, whose ends are positioned, once the operation has concluded, outside the lines of vaginal and retropubic suture, so that, acting on the vaginal or retropubic threads, it can be tightened/loosened with a view to eliminating any obstruction or, respectively, correcting the incontinence, if it persisted, being something that can be performed the day after the operation, with the patient awake, carrying out her typical activities, thus allowing any dysfunction caused from the sling implantation to be corrected.

### Brief description of the drawings

These and other characteristics and advantages of the invention will be more clearly shown from the detailed description below of a preferred embodiment, given only by way of illustrative and non-limitative example, with reference to the attached drawing, wherein
- Figure 1: shows side elevation and front elevation views of the mesh positioning element according to the invention, and
- Figure 2: is an illustrative representation of two alternative meshes, configured in accordance with the requirements of the system of the invention.

### Description of a preferred embodiment

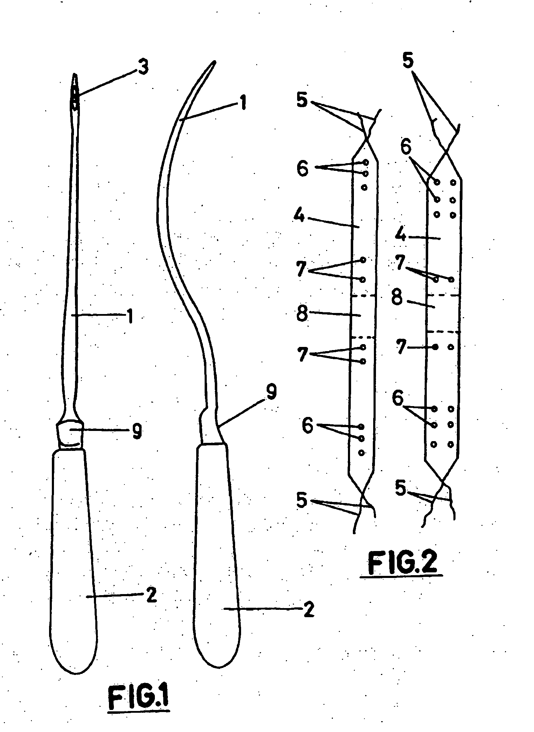

As stated above, the detailed description of the preferred embodiment of the invention will be made using the attached drawings, whereby numerical references will be made to designate the system components and the parts which define each one thereof. Thus, dealing firstly with the representation of Figure 1, we can observe different views corresponding to the side elevation and front elevation views of a mesh positioning device (or thread passer), configured so that therein we can distinguish a needle portion (1) and a handle portion (2). The needle portion (1), is elongated, with a predetermined length sufficient for the purpose for which it is intended, and it has been provided with a certain curve throughout, terminating in a blunt point at the distal end thereof. A through hole (3) has been provided in the proximity of this distal end, which is slightly elongated, coinciding with the longitudinal direction of the needle, and of considerable size, which can especially be observed in the front view of the positioning device. As will be understood, the positioning device can be constructed with the use of very varied materials which allow correct sterilization, said materials including both those of perishable and disposable type and reusable type.

Furthermore, the handle portion (2) is represented with a conventional form, ergonomically shaped to permit easy, comfortable gripping by the device user, provided with a depression (9) to support the thumb with a view to safer gripping, and of dimensions which facilitate the unit's handling.

Figure 2 shows the schematic representation of a portion of mesh of the type used as support in the correct positioning of the vesical neck and urethra. Said mesh portion is indicated with number (4), and consists of a band which, in its preferred embodiment, has an approximately rectangular shape, whose ends are narrowed and converge to terminate in filiform elements (5) at each one of its ends, whilst, on areas positioned around each end, and in other intermediate areas, said mesh portion has been provided with several respective holes (6, 7).

In accordance with both alternatives of embodiment of the mesh, the holes (6, 7) can be grouped in different forms, longitudinally or transversally, in rows and/or lines, depending on the number of holes made in accordance with the mesh width. This special feature can be easily observed in the examples illustrated in Figure 2, wherein, in the first alternative or mesh with less width, there are sets of three holes (6) beside the ends arranged longitudinally in rows, and sets of two holes (7) at both sides of an intermediate area (8), or urethral area, whilst in the second alternative or mesh with greatest width, we can observe sets of six holes beside both ends, arranged in two parallel rows of three holes each, whilst on both sides of the urethral area (8), two transversally aligned holes have been represented. Of course, as we have mentioned, the arrangement can be any other.

As will be understood, the inclusion of said holes (6, 7), permits the positioning device (1) to be applied with a view to pulling from any position, or even passing threads (not represented) through one or more of said holes, to be able to be then threaded through the hole (3) of the positioning device, and to be able to suitably adjust the positioning of the urethra which, in short, is the aim sought.

Preferably, two of the holes (7) of the mesh, will be arranged at approximately 1 and 2 cm from the urethral apposition area (8), whilst the other holes are arranged starting approximately five centimetres from the urethral area. The threads which pass through the holes (6), will be, once the operation has finished, positioned outside the vaginal and retropubic suture lines. As previously mentioned, by acting on the vaginal and retropubic lines, we can adjust the tension of the mesh to the most suitable tension for the patient's pathological characteristics. Furthermore, this form of mesh used by the system of the invention, also permits that the threads (5), projected from each end of the mesh (4), can be threaded in the hole (3) of the positioning device, so that it can be taken to its correct position.

As will be understood, the configuration adopted by the system components permit that, from the use of the latter, a series of advantages are derived, from which, those summarised below stand out as most important,:
- The procedure is less aggressive, by virtue of the needle used having a smaller calibre;
- The product is less expensive, since plastic covers are eliminated, simplifying the joining of the mesh to the thread passer, with the same needle serving for both approach directions;
- It is now possible to achieve what had not been attained up to now, and which consists of the modification of the tension given to the sling with the patient awake, therefore correcting any dysfunction (obstruction or incontinence) produced in the prior operation.

It is not considered necessary to expand on the content of this description for someone skilled in the art to understand its scope and the advantages derived from the invention, as well as to develop and put into practice the object thereof.

Nevertheless, it should be understood that the invention has been disclosed according to the preferred embodiment thereof, which means it can be modified without requiring any alterations of the essence of said invention, said modifications being able to affect, especially, the shape, size and/or manufacturing materials of the unit or any of the parts of the system, used.

The scope of the invention is defined by the appended claims.

## Claims

1. Improved system for the treatment of stress urinary incontinence, which provides means to return the vesical neck and the urethra to its original correct position with the use of a mesh which can be positioned beneath the urethra and tightened to a predetermined value corresponding with said desired position of the vesical neck and the urethra, wherein the system comprises:
A mesh positioning device which has a handle portion (2), ergonomically shaped to be easily and comfortably gripped, and a needle (1) appreciably curved throughout, and which, at its distal end, terminates in a blunt point, and has, beside this end, a wide slightly elongated through hole (3), extended in accordance with the longitudinal direction of the needle (1), and
a mesh element (4) in the form of an elongated band, of an optionally variable width, comprising a urethral area (8) in central position, said band narrowing at both ends; **characterized in that**
said mesh element (4) terminates at both ends in filiform elements (5) adapted to be threaded through the hole (3) of the needle (1) during the operations, and **in that** said mesh element comprises several holes in different positions,
comprising sets of holes (7) at each side of the urethral area (8) beside the urethral area and other sets of holes (6) positioned throughout the mesh element at each side of the urethral area beside both ends, said holes (6,7) of the mesh element being adapted to allow other threads to be threaded through them, said threads permitting the tightening/loosening of the mesh element (4) once positioned.

2. System according to claim 1, **characterized in that** two of the holes (7) beside the urethral area are positioned at a distance of approximately 1 and 2 cm respectively thereto.

## Patentansprüche

1. Verbessertes System zur Behandlung von Stress-Harninkontinenz, welches Mittel zum Zurückbringen des Blasenhalses und der Harnröhre in seine/ihre ursprüngliche korrekte Position vorsieht, mit der Verwendung einer Masche, welche unter der Harnröhre positioniert, und zu einem vorbestimmten der genannten gewünschten Position des Blasenhalses und der Harnröhre entsprechenden Wert festgezogen werden kann, wobei das System Folgendes umfasst:
eine Maschenpositionierungsvorrichtung, welche ein Handgriffteil (2) hat, welches ergonomisch geformt ist, um einfach und bequem gegriffen zu werden, und eine durchgehend deutlich gekrümmte Nadel (1), und welche an ihrem distalen Ende in einem stumpfen Punkt endet, und welche neben diesem Ende ein weites, leicht längliches Durchgangsloch (3) hat, welches sich gemäß der Längsrichtung der Nadel (1) erstreckt, und
ein Maschenelement (4) in Form eines länglichen Bandes, mit einer wahlweise veränderbaren Breite, welches einen Harnröhrenbereich (8) in zentraler Position umfasst, wobei sich das genannte Band an beiden Enden verengt; **dadurch gekennzeichnet, dass**
das genannte Maschenelement (4) an beiden Enden in fadenförmigen Elementen (5) endet, welche angepasst sind, um durch das Loch (3) der Nadel (1) während den Operationen gefädelt zu werden, und dass das genannte Maschenelement (4) mehrere Löcher in verschiedenen Positionen umfasst, welche Löchersätze (7) an jeder Seite des Harnröhrenbereichs (8) neben dem Harnröhrenbereich umfassen, und andere Löchersätze (6), die überall im Maschenelement an jeder Seite des Harnröhrenbereichs neben beiden Enden positioniert sind, wobei die genannten Löcher (6, 7) des Maschenelements angepasst sind, um zu ermöglichen, dass andere Fäden hindurch gefädelt werden, wobei die genannten Fäden das Festziehen/Lockern des Maschenelements (4) ermöglichen, nachdem es positioniert ist.

2. System nach Anspruch 1, **dadurch gekennzeichnet, dass** zwei der Löcher (7) neben dem Harnröhrenbereich jeweils mit einem Abstand von ungefähr 1 bzw. 2 cm bezüglich desselben positioniert sind.

## Revendications

1. Système amélioré pour le traitement de l'incontinence urinaire de stress, qui fournit des moyens pour renvoyer le cou de la vésicule et l'urètre à sa position correcte originelle en utilisant une maille qui peut être positionnée sous l'urètre et serrée à une valeur prédéterminée correspondant à ladite position désirée du cou de la vésicule et de l'urètre, dans lequel le système comprend :
un dispositif de positionnement de maille, qui a une partie de poignée (2) ergonomiquement conformée pour être facilement et aisément saisie, et une aiguille (1) sensiblement courbée dans toute sa longueur, et qui, à son extrémité distale, se termine en une pointe émoussée, et qui a à côté de cette extrémité, un large trou traversant légèrement allongé (3), qui s'étend selon la direction longitudinale de l'aiguille (1), et
un élément de maille (4) sous forme d'une bande allongée, d'une largeur optionnellement variable, comprenant une zone d'urètre (8) dans la position centrale, ladite bande s'étranglant sur les deux extrémités ; **caractérisé en ce que**
ledit élément de maille (4) se termine aux deux extrémités par des éléments filiformes (5) adaptés à être enfilés à travers le trou (3) de l'aiguille (1) pendant les opérations, et **en ce que** ledit l'élément de maille comprend divers tours dans différentes positions, comprenant des groupes de trous (7) de chaque côté de la zone d'urètre (8) à côté de la zone d'urètre et d'autres groupes de trous (6) positionnés à travers tout l'élément de maille de chaque côté de la zone d'urètre à côté des deux extrémités, lesdits trous (6, 7) de l'élément de maille étant adaptés pour permettre que d'autres filets se vissent à travers eux, lesdits filets permettant le serrage/desserrage de l'élément de maille (4) une fois positionné.

2. Système selon la revendication 1, **caractérisé en ce que** deux des trous (7) à côté de la zone d'urètre sont positionnés à une distance d'environ 1 et 2 cm respectivement par rapport à celle-ci.
